# EUROPEAN PATENT APPLICATION

(11) **EP 4 582 028 A1**
(43) Date of publication of application: **09.07.2025**
(21) Application number: 24150032.1
(22) Date of filing: 02.01.2024
(51) Int. Cl.: A61B 7/00, G10L 25/66

(54) **ACOUSTIC MONITORING SYSTEM, METHOD, COMPUTER PROGRAM PRODUCT, AND COMPUTER-READABLE STORAGE MEDIUM**

(71) Applicant: Koninklijke Philips N.V., 5656 AG Eindhoven (NL)
(72) Inventor: DEN BRINKER, Albertus Cornelis, Eindhoven (NL); OUWELTJES, Okke, Eindhoven (NL); RIETMAN, Ronald, 5656AG Eindhoven (NL); MORICE, Alyn Hugh, Eindhoven (NL); CROOKS, Michael, Eindhoven (NL)
(74) Representative: Philips Intellectual Property & Standards

(57) **Abstract**

There is provided a method of acoustic monitoring. The method comprises receiving acoustical data obtained in the vicinity of a subject; identifying a first portion of the acoustical data representing acoustic events; identifying a second portion of the acoustical data representing no acoustic events; providing samples based on the first portion of the acoustical data to a classifier; and creating classified samples. Each classified sample is classified by the classifier as a first class or a second class. The method comprises determining a property of at least one of i) the second portion, and ii) the classified samples; and performing a comparison of the property with a reference property. The reference property is i) acoustical training data used to train the classifier during a training phase, or ii) at least one classified training sample created by the classifier based on the training acoustical data during the training phase.

## Description

### FIELD OF THE INVENTION

The invention relates to a method of acoustic monitoring of a subject, in particular acoustic monitoring of acoustic events related to a condition of the respiratory system of the subject, such as coughing events. Further, the invention relates to an acoustic monitoring system comprising a processing system to perform the method of acoustic monitoring. Further, the invention relates to a computer program and a computer-readable medium, comprising instructions which, when executed by a processor, cause the processing system to carry out the method.

### BACKGROUND OF THE INVENTION

Many people suffer from one or more conditions of the respiratory system. These conditions include chronic obstructive pulmonary disease (COPD), sleep apnea and asthma. People with such a condition may be monitored to determine changes in the severity of the condition. For example, subjects with COPD may suffer from exacerbations, which are an acute worsening of the respiratory symptoms. Accurate monitoring helps to provide proper medical care quickly to counter the worsening of the condition.

Some conditions of the respiratory system cause the subject to generate sound, such as coughing, wheezing, or snorting. These acoustic events provide information about the severity of the condition. A change of the severity of the condition results in a change of the acoustic events.

It is known to monitor such acoustic events with microphones. A microphone allows for unobtrusive and longitudinal monitoring of the subject. The microphone provides acoustical data to a processing system that determines the presence of the acoustic events. Based on the presence of the acoustic events, the severity of the condition is estimated.

However, the microphone does not only receive sound created by the subject as a result of the condition, but also other sounds. The microphone may receive other sounds created by the subject, such as talking or moving, and may receive sound created by the environment, such as traffic, home appliances, or pets. Classifiers have been developed that determine whether the sound received by the microphone represents acoustic events related to the condition.

### SUMMARY OF THE INVENTION

The known classifiers are optimized to classify sound as an acoustic event for a certain environment to provide accurate optimization. The known classifiers are therefore optimized to distinguish the acoustic events caused by the condition from other sounds in that certain environment. However, the environment in which the acoustic monitoring takes place may change. For example, the monitoring takes place in a different location, the subject watches tv during monitoring, or a nearby window is opened which increases the level of street noise received by the microphone. The known classifiers may provide unreliable results after such a change in the environment. There is no indication that the results may be unreliable.

It is an objective of the invention to provide improved acoustic monitoring.

According to a first aspect of the invention, there is provided a method acoustic monitoring of a subject, the method comprising;
receiving acoustical data obtained in the vicinity of the subject;
identifying a first portion of the acoustical data representing acoustic events;
identifying a second portion of the acoustical data representing no acoustic events;
providing samples based on the first portion of the acoustical data to a classifier;
creating classified samples by classifying the samples with the classifier,
wherein each classified sample is classified by the classifier as a first class or a second class,
wherein the first class represents a respiratory acoustic event related to a condition of the respiratory system of the subject,
wherein the second class does not represent the respiratory acoustic event;
determining a property of at least one of
   i) the second portion of the acoustical data, and
   ii) the classified samples;
performing a comparison of the property with a reference property,
wherein the reference property is of at least one of
   i) acoustical training data used to train the classifier during a training phase, and
   ii) at least one classified training sample created by the classifier based on the training acoustical data during the training phase; and
generating a similarity metric based on the comparison, wherein the similarity metric is representative of similarity between the property and the reference property.

The inventors have realized that a change in the environment is observable via the acoustical data or via the classification done by the classifier based on the acoustical data by comparing with a reference property of the acoustical training data or the classified training samples. In case a property of the second portion of the acoustical data differs substantially from the reference property of the acoustical training data, the input for the classifier is based on acoustical data for which the classifier is not properly trained. A change in the environment causes a difference in the acoustical data. The difference in the acoustical data causes a difference between the classified samples and the classified training samples. Therefore, this difference between the classified samples and the classified training samples is a measure of a change in the environment. In case a property of the classified samples differs substantially from the classified training samples, it is likely that the classifier performs unreliably as a result of the change in environment.

By comparing the property with the reference property, the similarity metric is generated. In case the similarity metric indicates a high level of similarity between the property and the reference property, the classification of the classifier is reliable, because the classifier classifies based on acoustical data for which the classifier is trained. In case the similarity metric indicates a low level of similarity between the property and the reference property, the classification of the classifier is unreliable, because the classifier classifies based on acoustical data for which the classifier is not trained or is not properly trained. As a result, the similarity metric provides an insight into the reliability of the classification. A care professional may take the similarity metric into account when assessing the acoustic monitoring. In case the similarity metric indicates a low level of similarity, a care professional may decide to repeat the acoustic monitoring, to retrain the classifier or to provide an alternative monitoring. As a result, improved acoustic monitoring is obtained of the subject suffering from a condition of the respiratory system.

According to a second aspect of the invention, there is provided a computer program product, comprising instructions which, when executed by a processor, cause the processing system to carry out the method of the first aspect of the invention.

According to a third aspect of the invention, there is provided an acoustic monitoring system, comprising:
a processing system configured to perform the method of the first aspect of the invention; and
an acoustic sensor for generating the acoustical data representative of acoustic events related to the condition of the respiratory system of the subject,
wherein the processing system is coupled to the acoustic sensor to receive the acoustical data.

According to a fourth aspect of the invention, there is provided a computer-readable storage medium comprising instructions which, when executed by a processing system, cause the processing system to carry out the method of the first aspect of the invention.

### BRIEF DESCRIPTION OF THE DRAWINGS

Exemplary embodiments will now be described, by way of example only, with reference to the following figures, in which:
FIG. 1 depicts a first embodiment according to the invention;
FIG. 2 depicts a detail of the first embodiment according to the invention;
FIG. 3 depicts the processing system according to the first embodiment;
FIG. 4 depicts acoustical data as provided by the acoustic sensor, in the first embodiment;
FIG. 5 depicts two spectral tilts;
FIG. 6 depicts the processing system according to a second embodiment;
FIG. 7 depicts a distribution of probabilities of classified samples according to the second embodiment;
FIG. 8 depicts a distribution of probabilities of classified samples according to a third embodiment of the invention;
FIG. 9 depicts a fourth embodiment of the invention;
FIG. 10 depicts a fifth embodiment of the invention, which may be implemented in addition to the fourth embodiment.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

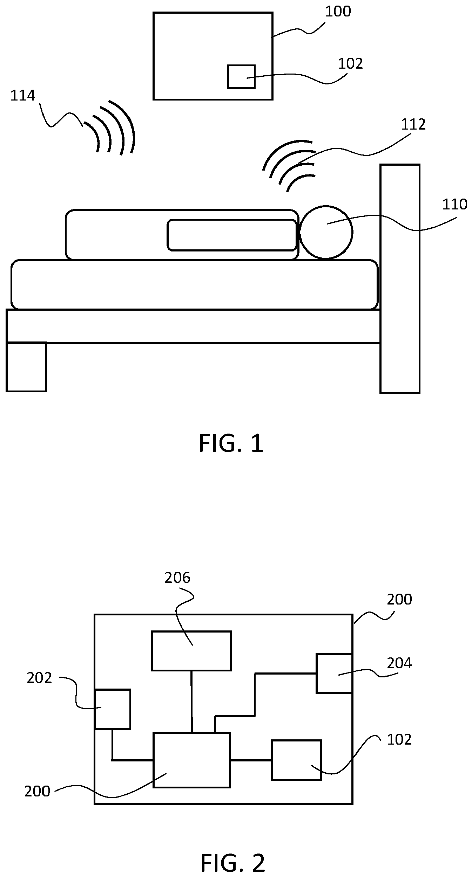

FIG. 1 depicts a first embodiment according to the invention. There is provided an acoustic monitoring system 100. The acoustic monitoring system 100 comprises an acoustic sensor 102 for generating the acoustical data representative of acoustic events related to the condition of the respiratory system of the subject 110. The subject 110 may suffer from a condition of the respiratory system such as COPD, sleep apnea or asthma. As a result of this condition, the subject 110 makes sounds such as coughing or wheezing or snorting. The acoustic monitoring system 100 is located in the vicinity of the subject 110, for example next to the bed of the subject 110 on a nightstand. The acoustic sensor 102, such as a microphone, is able to receive the sound 112 from the subject 110. While the acoustic monitoring system 100 is monitoring the sounds 112 of the subject 110, other sounds 114 may be received by the acoustic sensor 102. These sounds 114 may be generated by traffic, or by pets or by other people in the vicinity of the subject 110. The acoustic monitoring system 100 is configured to distinguish between the sounds 112 made by the subject 110 as a result of the condition of the respiratory system, and the other sounds 114, as will be explained further below.

The acoustic monitoring system 100 monitors the subject 110 during a monitoring session. The goal of the monitoring session is to obtain information about the condition of the respiratory system of the subject 110 based on the sounds 112 received by the acoustic monitoring system 100.

FIG. 2 depicts a detail of the first embodiment according to the invention. The acoustic monitoring system 100 comprises the acoustic sensor 102, a processing system 200, an input 202, an output 204 and a memory 206. The processing system 200 is coupled to the acoustic sensor 102 to receive the acoustical data. The processing system 200 configured to perform the method of any one of the embodiments described below.

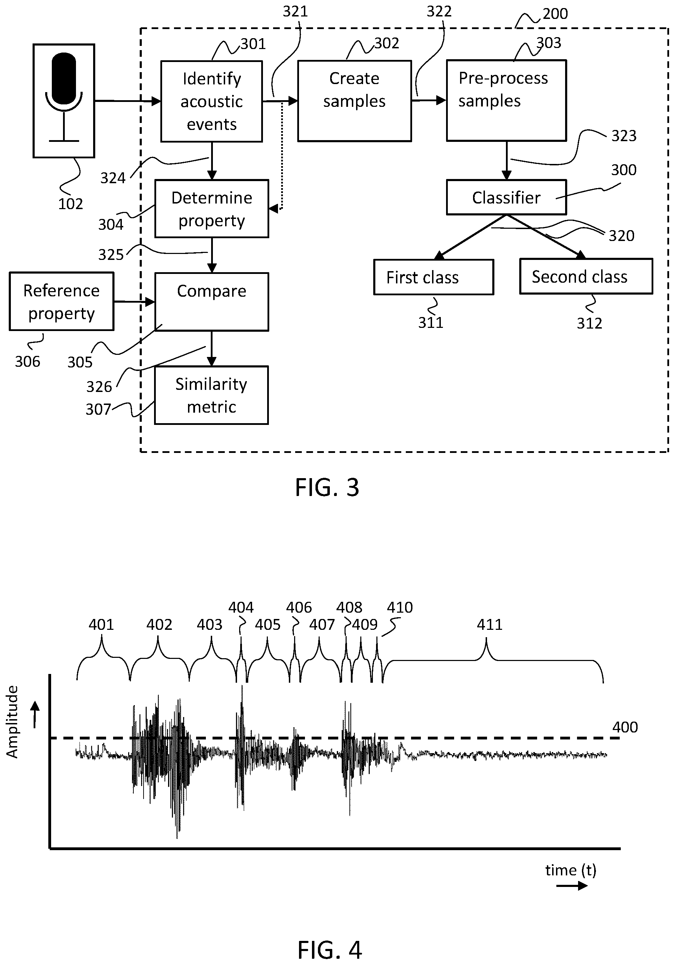

FIG. 3 depicts the processing system 200 according to the first embodiment. The processing system 200 comprises a classifier 300. The processing system 200 is configured to perform the following method. The processing system 200 is configured to receive acoustical data obtained in the vicinity of the subject 110. The processing system 200 is configured to identify a first portion 321 of the acoustical data representing acoustic events, and to identify a second portion 324 of the acoustical data representing no acoustic events. The processing system 200 is configured to provide samples 322 based on the first portion 321 of the acoustical data to the classifier 300. The processing system 200 is configured to create classified samples 320 by classifying the samples 322 with the classifier 300. Each classified sample 320 is classified by the classifier 300 as a first class 311 or a second class 312. The first class 311 represents a respiratory acoustic event related to a condition of the respiratory system of the subject 110. The second class 312 does not represent the respiratory acoustic event. The processing system 200 is configured to determine a property 325 of at least one of i) the second portion 324 of the acoustical data, and ii) the classified samples 320. The processing system 200 is configured to perform a comparison of the property 325 with a reference property 306. The reference property 306 is of at least one of i) acoustical training data used to train the classifier 300 during a training phase, and ii) at least one classified training sample created by the classifier 300 based on the training acoustical data during the training phase. The processing system 200 is configured to generate a similarity metric 307 based on the comparison 326. The similarity metric 307 is representative of similarity between the property 325 and the reference property 306.

In the training phase of the classifier 300, the classifier 300 is trained to recognize acoustic events relating to the condition of the respiratory system of the subject 110, and to correctly classify such acoustic events as the first class 311. The training phase comprises training the classifier 300, testing the classification done by the classifier 300 based on the training, and validating the classification.

The classifier 300 comprises, for example, a linear classifier, or a quadratic classifier, or a support vector machine, or decision tree learning such as random forest, or an artificial neural network. The classifier is, for example, a binary classifier with features of the samples 322 as input and a number between 0 and 1, treated as a probability, as output. By thresholding the number the class is provided, i.e., either the first class 311 or the second class 312. The sensitivity and specificity of the classifier 300 can be controlled by setting the threshold.

The processing system 200 receives the acoustical data from the acoustic sensor 102. In step 301, the processing system 200 identifies a first portion 321 of the acoustical data representing acoustic events, and identifies a second portion 324 of the acoustical data representing no acoustic events. For example, the processing system 200 checks which portions of the acoustical data represent a sound level exceeding a threshold, and/or represent a change in sound level, and/or represent sounds with a certain frequency content to assign these portions to the first portion 321. The other portions of the acoustical data are assigned to the second portion 324. The first portion 321 does not have to be a single continuous portion, but may be a plurality of portions separated in time by a plurality of second portions 324. More explanation is shown in FIG. 4.

FIG. 4 represents the acoustical data as provided by the acoustic sensor 102. The acoustical data is provided via an electric signal, in this example. The figure shows the amplitude of the electric signal as a function of time. The amplitude depends on the sound level of the sound received by the acoustic sensor 102, wherein a large amplitude corresponds to a high sound level. In an embodiment, the processing system 200 is configured to compare the amplitude with an amplitude threshold 400. The portions of the acoustical data that correspond to an amplitude that exceeds the amplitude threshold 400, are assigned to the first portion 321. The portions of the acoustical data that correspond to an amplitude that does not exceed the amplitude threshold 400, are assigned to the second portion 324. The portions 402, 404, 406, 408, and 410 of the acoustical data exceed the amplitude threshold 400, and are therefore assigned by the processing system 200 to the first portion 321. The portions 401, 403, 405, 407, 409, and 411 of the acoustical data do not exceed the amplitude threshold 400, and are therefore assigned by the processing system 200 to the second portion 324. In addition or alternatively to comparing the amplitude with the amplitude data 400, the processing system 200 may use other methods to detect an acoustic event in the acoustical data and assigning such an acoustic event to the first portion 321. For example, the processing system 200 detects an acoustic event based on a change in sound level of the acoustical data, or a frequency content of the acoustical data.

Depending on the sounds 112 made by the subject 110, and the other sounds 114 made in the environment, the first portion 321 may be substantially smaller than the second portion 324. For example, the total cumulative duration of the acoustic events during a monitoring session that are represented by the first portion 321 may be less than 10% or less than 5% or less than 1% of the total cumulative duration of the second portion 324. This may be the case for a subject 110 that coughs occasionally in an otherwise silent environment. In such a case, it may be that the portion 411 extends for an hour or more, whereas the duration of the portions 401-410 extend for only a few seconds. The total cumulative duration of the first portion 321 may be higher, for example, in case the subject 110 coughs frequently while in a noisy environment.

Returning to FIG. 3, the processing system 200 is configured to provide samples 322 based on the first portion 321 of the acoustical data to a classifier 300. First, the processing system 200 creates, in step 302, samples 322 based on the first portion 321 of the acoustical data. So the portion of the acoustical data in which acoustic events are present is used to create the samples 322. Then, in step 303, the samples 322 are pre-processed. For example, in step 303, features are extracted from the samples 322, and/or derivatives of the samples 322 are obtained. After the pre-processing in step 303, the pre-processed samples 323 are provided to the classifier 300.

In an embodiment, step 303 of pre-processing the samples 322 is omitted. In an embodiment, step 303 of pre-processing the samples 322 is included in step 302 when creating the samples 322. In an embodiment step 303 of pre-processing the samples 322 is performed by the classifier 300.

The classifier 300 creates classified samples 320 by classifying the samples 322 with the classifier 300. Each classified sample 320 is classified by the classifier 300 as a first class 311 or a second class 312. The first class 311 represents a respiratory acoustic event related to the condition of the respiratory system of the subject 110. The second class 312 does not represent the respiratory acoustic event. Optionally, the classifier 300 is configured to creates classified samples 320 by classifying the samples 322 in more classes than just the first class 311 and the second class 312. For example, the classifier 300 may make use of one or more further classes. For example, further classes may be used to classify other types of acoustic events. For example, a further class may be used to classify environmental sound events. For example, a further class may be used to classify other sound events caused by the subject 110, such as talking. For example, the classifier 300 may subdivide the first class 311 and/or the second class 312 into further classes.

The classifier 300 receives the samples 322. The samples 322 represent acoustic events. These acoustic events include acoustic events related to the condition of the respiratory system of the subject 110, but also includes other acoustic events, such as environmental sounds 114. The classifier 300 is configured to recognize the acoustic events related to the condition of the respiratory system of the subject 110. For example, the classifier 300 is configured to recognize coughing of the subject 110. For each sample, the classifier 300 determines whether a sample 322 represents a cough or does not represent a cough. If the sample 322 represents a cough, the sample 322 is classified in the first class 311. If the sample 322 does not represent a cough, the sample 322 is classified in the second class 312. For each sample 322, the classifier 300 determines whether a sample 322 represents wheezing or does not represent wheezing. If the sample 322 represents wheezing, the sample 322 is classified in the first class 311. If the sample 322 does not represent wheezing, the sample 322 is classified in the second class 312.

For example, the condition of the respiratory system comprises coughing. The first class 311 represents a coughing event. The second class 312 does not represent a coughing event.

The processing system 200 is, for example, configured to generate an output signal representing the classified samples 320. The output signal is, for example, sent to the output 204. The output signal is, for example, further used to generate an image on a display to represent the outcome of the acoustic monitoring based on the classified samples 320.

The processing system 200 is further configured to determine a property 325 of at least one of the second portion 324 of the acoustical data in step 304. In known methods using classifiers, the second portion 324 of the acoustical data is discarded, because the second portion 324 does not represent acoustical events, and the second portion 324 is therefore not used to create samples. However, according to this embodiment of the invention, the second portion 324 of the acoustical data is used to determine the similarity metric 307, as is explained further.

The property 325 is, for example, an average sound level of the second portion 324 or an average sound level of both the first portion 321 and the second portion 324. To quantify the average sound level, various scales are known, which are typically on a logarithmic scale. The scales reflect the sound pressure or acoustic pressure or loudness. Loudness is the subjective perception of sound pressure, e.g., dB(A), dB(B), dB(C). The average sound level is, for example, low in case the acoustic monitoring of the subject 110 takes place in a silent environment. The average sound level is, for example, high in case the acoustic monitoring of the subject 110 takes place in an environment with a noisy air conditioner, heavy traffic, or when the subject 110 watches tv or listens to music. The processing system 200 may determine the average sound level based on both the second portion 324 and the first portion 321.

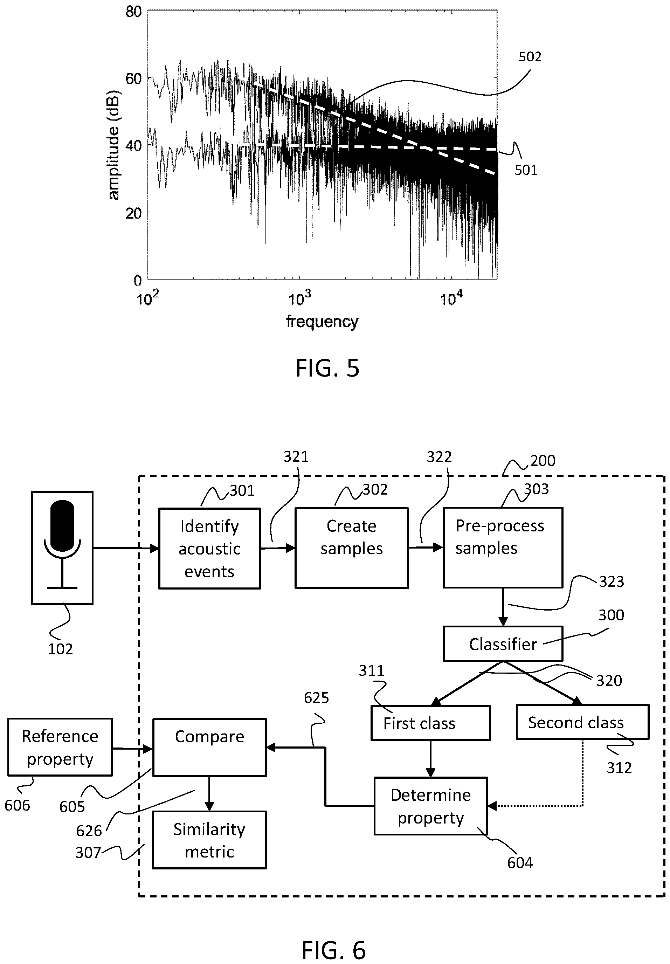

The property 325 is, for example, spectral tilt. Sound is not only characterized by the sound level but also by a spectral content. The balance between low and high spectral contents can be captured in a quantity like spectral tilt. Spectral tilt quantifies the overall slope of the power spectral density. The concept of a spectral slope is illustrated in the FIG. 5. Line 501 indicates a spectral content of sound with a constant slope of the power spectral density for frequencies of 400 Hz and higher. Line 502 indicates a spectral content of sound a declining slope of the power spectral density for frequencies of 400 Hz and higher. The processing system 200 may determine the spectral tilt based on the second portion 324 or both the second portion 324 and the first portion 321.

The property 325 is, for example, a number of acoustic events. Based on information from the second portion 324, the processing system 200 is able to determine how many acoustic events occurred. For example, the number of acoustic events is determined by the processing system 200 based on the number of portions of the acoustical data that form the second portion 324. In the example of FIG. 4, the processing system 200 may count the portions 401, 403, 405, 407, 409 and 411 to determine the number of acoustic events. In another example, the processing system 200 determines the number of acoustic events based on both the first portion 321 and the second portion 324.

The property 325 is, for example, a temporal distance between acoustic events. Based on information from the second portion 324, the processing system 200 is able to determine how much time there is between acoustic events. The processing system 200 may determine the temporal distance between acoustic events based on both the second portion 324 and the first portion 321.

The processing system 200 determines the property 325, or multiple properties, of the second portion 324 of the acoustical data. The processing system 200 is configured to receive the reference property 306. The reference property 306 is a characteristic of the acoustical training data used to train the classifier 300 during the training phase. The acoustical training data comprises a first portion and a second portion. The first portion of the acoustical training data is representative of acoustic events. The second portion of the acoustical training data is representative of no acoustic events. The reference property 306 is based on a characteristic of at least the second portion of the acoustical training data.

For example, the property 325 is the average sound level of the second portion 324 of the acoustical data during a monitoring session, and the reference property 306 is the average sound level of the second portion of the acoustical training data during the training phase. For example, the property 325 is the spectral tilt of the second portion 324 of the acoustical data during a monitoring session, and the reference property 306 is the spectral tilt of the second portion of the acoustical training data during the training phase. For example, the property 325 is the number of acoustic events of the second portion 324 of the acoustical data during a monitoring session, and the reference property 306 is the number of acoustic events of the second portion of the acoustical training data during the training phase. For example, the property 325 is the temporal distance between acoustic events during a monitoring session, and the reference property 306 is the temporal distance between acoustic events during the training phase during the training phase.

In step 305, the processing system 200 performs the comparison of the property 325 with the reference property 306. For example, the processing system 200 compares the average sound level during the monitoring session with the average sound level during the training phase. For example, the processing system 200 compares the spectral tilt during the monitoring session with the spectral tilt during the training phase. For example, the processing system 200 compares the number of acoustic events during the monitoring session with the number of acoustic events during the training phase. For example, the processing system 200 compares the temporal distance between acoustic events during the monitoring session with the temporal distance between acoustic events during the training phase.

In step 307, the processing system 200 generates the similarity metric 307 based on the comparison. The similarity metric 307 is representative of similarity between the property 325 and the reference property 306. For example, the similarity metric 307 provides a binary result, indicating whether the comparison indicates that the difference between the property 325 and the reference property 306 exceeds a threshold or not. For example, the similarity metric 307 is mapped to a scale, such as a color scale, indicating the similarity based on the comparison. For example, the scale is a category scale. For example, the similarity metric 307 provides a single metric for an entire monitoring session. For example, the similarity metric 307 provides multiple metrics for a monitoring session, such as a metric for every hour of the monitoring session or any other time interval.

FIG. 6 depicts the processing system 200 according to a second embodiment. The second embodiment is, for example, the same as the first embodiment, except for the following.

The processing system 200 receives the acoustical data from the acoustic sensor 102. In step 301, the processing system 200 identifies a first portion 321 of the acoustical data representing acoustic events, and identifies a second portion 324 of the acoustical data representing no acoustic events. For example, the processing system 200 checks which portions of the acoustical data represent a sound level exceeding a threshold, and/or represent a change in sound level, and/or sounds with a certain frequency content to assign these portions to the first portion 321. The other portions of the acoustical data are assigned to the second portion 324. The first portion 321 does not have to be a single continuous portion, but may be a plurality of portions separated in time a plurality of second portions, as is shown in FIG. 4.

Returning to FIG. 6, the processing system 200 is configured to provide samples based on the first portion 321 of the acoustical data to a classifier 300. First, the processing system 200 creates, in step 302, samples based on the first portion 321 of the acoustical data. So the portion of the acoustical data in which acoustic events are present is used to create the samples 322. Then, in step 303, the samples 322 are pre-processed. For example, in step 303, features are extracted from the samples 322, and/or derivatives of the samples 322 are obtained. After the pre-processing in step 303, the pre-processed samples 323 are provided to the classifier 300.

In an embodiment, step 303 of pre-processing the 322 is omitted. In an embodiment step 303 of pre-processing the samples 322 is included in step 302 when creating the samples 322. In an embodiment step 303 of pre-processing the samples 322 is performed by the classifier 300.

The classifier 300 creates classified samples 320 by classifying the samples 322 with the classifier 300. Each classified sample 320 is classified by the classifier 300 as a first class 311 or a second class 312. The first class 311 represents a respiratory acoustic event related to the condition of the respiratory system of the subject 110. The second class 312 does not represent the respiratory acoustic event.

The classifier 300 receives the samples 322. The samples 322 represent acoustic events. These acoustic events include acoustic events related to the condition of the respiratory system of the subject 110, but also includes other acoustic events, such as environmental sounds 114. The classifier 300 is configured to recognize the acoustic events related to the condition of the respiratory system of the subject 110. For example, the classifier 300 is configured to recognize coughing of the subject 110. For each sample, the classifier 300 determines whether that sample 322 represents a cough or does not represent a cough. If the sample 322 represents a cough, the sample 322 is classified in the first class 311. If the sample 322 does not represent a cough, the sample 322 is classified in the second class 312. For each sample 322, the classifier 300 determines whether that sample 322 represents wheezing or does not represent wheezing. If the sample 322 represents wheezing, the sample 322 is classified in the first class 311. If the sample 322 does not represent wheezing, the sample 322 is classified in the second class 312.

The processing system 200 is configured to determine, in step 604, a property 625 of the classified samples 320. The processing system 200 is configured to perform a comparison of the property 625 with a reference property 606. The reference property 606 is of at least one classified training sample created by the classifier 300 based on the training acoustical data during the training phase.

The processing system 200 creates with the classifier 300 the classified samples 320 with a probability of being the first class 311. The classifier 300 classifies a sample 322 as the first class 311 if the probability is above a threshold, and classifies a sample 322 as the second class 312 if the probability is below the threshold. The processing system 200 determines the property 625 based on the probability of at least one classified sample 320. The at least one classified sample 320 may be at least one classified sample 320 of the first class 311, or at least one classified sample 320 of the second class 312, or a plurality of classified samples 320 in the first class 311 and the second class 312.

For example, during the training phase, the classifier 300 classifies classified training samples based on acoustical training data with a probability of being the first class 311. The classifier 300 classifies a training sample as the first class 311 if the probability is above the threshold, and classifies a training sample as the second class 312 if the probability is below the threshold.

For example, the reference property 606 is based on the probability of at least one classified training sample. The at least one classified training sample may be at least one classified training sample of the first class 311, or at least one classified training sample in the second class 312, or a plurality of classified training samples in the first class 311 and the second class 312.

For example, the processing system 200 performs in step 605, a comparison of the property 325 of the classified samples 320 with the reference property 606 of the classified training samples.

The processing system 200 generates the similarity metric 307 based on the comparison, wherein the similarity metric 307 is representative of similarity between the property 625 of the classified samples 320 and the reference property 606 of the classified training samples.

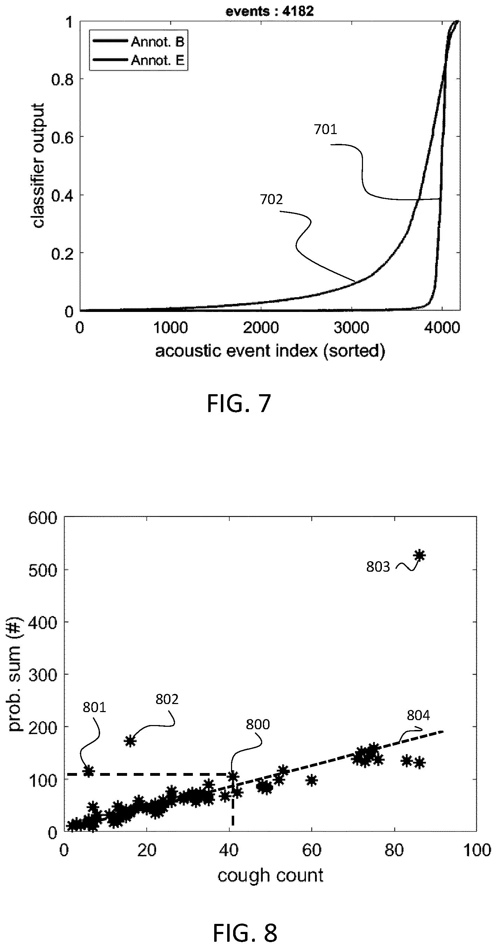

In an embodiment, the property 625 is based on a distribution of the probabilities of at least a subset of the classified samples 320, which is illustrated in FIG. 7. FIG. 7 shows on the x-axis 4182 classified samples 320 of a monitoring session. The y-axis shows the probability of each classified sample 320 has of being in the first class 311. The probability is a number from 0 to 1. The classified samples 320 are sorted based on the value of their probability. Classified sample #1 has the lowest probability of being a first class 311, whereas classified sample #4182 has the highest probability of being a first class 311. Line 701 represents the distribution of the probability for the classified samples 320 of the training phase. Line 701 indicates a well-trained classifier 300, because most classified samples 320 either have a very low probability, e.g. below 0.05, or a very high probability, e.g., above 0.8. This means that the classifier 300 is able to reliably classify the classified samples 320 with the very low probability as the second class 312, and the classified samples 320 with the very high probability as the first class 311. Only a few classified samples 320 do not have a very low probability or a very high probability. The classification of these few classified samples 320 may be unreliable. However, since there are only a few of the unreliable classified samples, the acoustic monitoring is not significantly affected.

Line 702 represents the probability for the classified samples 320 during a monitoring session. As is clear from line 702, the monitoring session has fewer classified samples 320 with a very low probability and fewer classified samples 320 with a very high probability. Line 702 indicates a larger number of classified samples 320 that have a probability between 0.05 and 0.8 than the classified samples 320 of the training session. For the classified samples 320 with a probability between 0.05 and 0.8, it is less sure whether these classified samples 320 are a first class 311 or a second class 312. In case the number of classified samples 320 with a probability between 0.05 and 0.8 exceeds a threshold, the classifier 300 may provide unreliable classification caused by a difference in the environment of the subject 110.

As an example, the values of 0.05 and 0.8 are used for respectively a very low probability and a very high probability. In a different embodiment, any other suitable numbers may be used for the threshold representing a very low probability, indicating a classified sample 320 is highly likely a second class 312, and the threshold representing a very high probability, indicating a classified sample 320 is highly likely a first class 311. For example, the values are 0.01 and 0.9, or 0.1 and 0.7, or 0.2 and 0.8.

As shown in FIG. 7, the property 625 is based on a distribution of probabilities of the classified training samples, i.e., line 702. The reference property 606 is based on the distribution of probabilities of the classified training samples, i.e., line 701.

FIG. 8 depicts a third embodiment of the invention. The third embodiment is, for example, the same as the first embodiment and/or as the second embodiment, except for the following.

The processing system 200 receives multiple sets of acoustical data. Each set of acoustical data represents a different time interval. For example, each set of acoustical data represents a monitoring session. For example, each set of acoustical data represents a monitoring session of a different day. For example, each monitoring session lasts in the range of 4-12 hours.

For each set of acoustical data, the classifier 300 creates classified samples 320. For each set of acoustical data, the processing system 200 sums the probability of the classified samples 320, and counts a number of classified samples 320 in the first class 311. The x-axis of FIG. 8 shows the number of classified samples 320 in the first class 311 for each set of acoustical data. The y-axis of FIG. 8 shows the sum of the probabilities of all classified samples 320 for each set of acoustical data. The sum of the probabilities includes the probabilities of classified samples 320 in both the first class 311 and the second class 312. To give an example, one value 800 indicates for a specific set of acoustical data, that there are 41 classified samples 320 in the first class 311. The sum of the probability of the classified samples 320 in that set of acoustical data is 102.

The processing system 200 is configured to determine a correlation 804 between the number of classified samples 320 in the first class 311 and the sum of the probability, based on the multiple sets of acoustical data.

FIG. 8 is based on the same 4182 samples as FIG. 7. Ideally, in case the classifier 300 classifies 41 classified samples 320 as the first class 311, the sum of the probabilities of all classified samples 320 is: 41 times a probability of 1 for the classified samples 320 in the first class 311, and 4141 times a probability of 0 for the other classified samples 320 in the set of acoustical data. However, in reality, the classified samples 320 in the second class 312 have some probability that they are first class 311. This probability is low, for example between 0.01 and 0.05, but because of the large number of classified samples 320 in the second class 312, the sum of probabilities is 102 for that set of acoustical data. The inventors have realized that the sum of probabilities scales with the number of classified samples 320 in the first class 311. As indicated by the correlation 804, there is a linear correlation.

The processing system 200 determines as the property 625 a deviation between the sum of the probabilities and the correlation 804 for each set of acoustical data. As can be seen in FIG. 8, most values in the graph are near the correlation 804. However, the three points 801-803 deviate by a large amount. So for the monitoring sessions in which those sets of acoustical data are obtained, the classification may be unreliable. The subject 110 and/or the caretaker may investigate what happened during those monitoring sessions. It may be that the subject 110 was monitored at a different location than normal, or that there were environmental sounds 114 that are normally not present.

The embodiments described above may be combined in a single implementation of the invention. For example, the processing system 200 is configured to generate the similarity metric 307 based on a comparison of a property 325 of the second portion 324 of the acoustical data as described in the first embodiment, and based on a comparison of a property 625 of the classified samples 320 as described in the second embodiment and/or the third embodiment.

In an embodiment, in case the similarity exceeds a similarity threshold, the similarity metric 307 indicates a change in an environment of the subject 110 between when the acoustical data was obtained and when the acoustical training data was obtained.

The similarity metric 307 may suggest to retrain the classifier 300 for the change in the environment, in case the similarity exceeds the similarity threshold. For example, in case the spectral tilt during multiple monitoring session differs substantially from the spectral tilt during the training phase, the similarity metric 307 may suggest retraining the classifier 300. For example, in case the average sound level during multiple monitoring sessions differs substantially from the average sound level during the training phase, the similarity metric 307 may suggest to retrain the classifier 300. For example, in case the line 701 differs substantially from the line 702 during multiple monitoring sessions, the similarity metric 307 may suggest to retrain the classifier 300. For example, a substantial number of values in FIG. 8 differ substantially from the correlation 804, the similarity metric 307 may suggest to retrain the classifier 300.

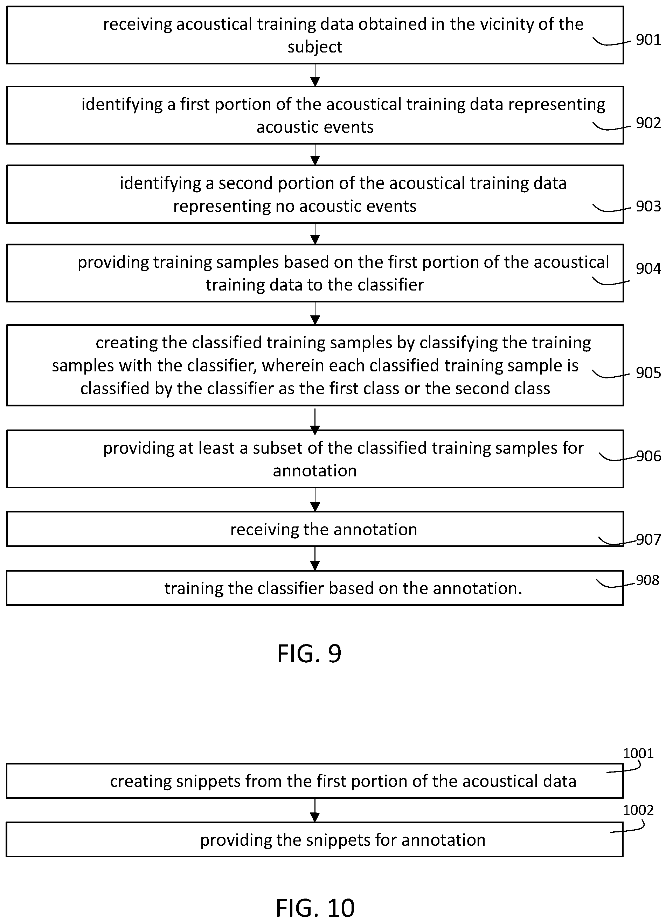

FIG. 9 depicts a fourth embodiment of the invention. The fourth embodiment is, for example, included in any one of the first embodiment, the second embodiment and the third embodiment.

In the fourth embodiment, the processing system 200 is configured to perform the following method. In step 901, the processing system 200 receives acoustical training data obtained in the vicinity of the subject 110. In step 902, the processing system 200 identifies a first portion of the acoustical training data representing acoustic events. In step 903, the processing system 200 identifies a second portion of the acoustical training data representing no acoustic events. In step 904, the processing system 200 provides training samples based on the first portion of the acoustical training data to the classifier 300. In step 905, the classifier 300 creates the classified training samples by classifying the training samples, wherein each classified training sample is classified by the classifier 300 as the first class 311 or the second class 312. In step 906, the processing system 200 provides at least a subset of the classified training samples for annotation. In step 907, the processing system 200 receives the annotation. In step 908, the processing system 200 trains the classifier 300 based on the annotation.

Preferably, the acoustical training data is obtained in the same environment in which the subject 110 will use the acoustic monitoring system 100 during monitoring sessions. For example, the acoustical training data is obtained in the bedroom of the subject 110, and the monitoring session is also obtained in the bedroom.

The classified training samples are provided for annotation. For example, the processing system 200 is configured to send the classified training samples to the output 204. Via the output 204, the classified training samples are, for example, sent to an external device. A professional annotator may use the external device to annotate the classified training samples either manually or automatically. This means that the professional annotator indicates for the classified training samples whether a classified training sample is a first class 311 or a second class 312. The professional annotator, for example, indicates whether a classified training sample represents a cough or not a cough. Via the external device, the professional annotator provides the annotation back to the processing system 200. Based on the annotation, the processing system 200 is configured to train or retrain the classifier 300 to improve the performance of the classifier 300.

FIG. 10 depicts a fifth embodiment of the invention, which may be implemented in addition to the fourth embodiment. The fifth embodiment is a method comprising in step 1001 creating snippets from the first portion of the acoustical training data, and in step 1002 providing the snippets for annotation.

A snippet is a short sound sample that has an acoustic event. By creating the snippets and providing the snippets for annotation, the professional annotator is able to provide an improved annotation of the classified samples, for example, because the professional annotator is able to hear the acoustic event corresponding to the classified sample. For example, the professional annotator is able to perform additional sound analysis of the snippet to improve the annotation. The snippets prevent that whole conversations in the vicinity of the subject 110 are provided to the professional annotator, which helps to safeguard the privacy of the subject 110.

In an embodiment, there is provided a computer program product, comprising instructions which, when executed by the processing system 200, cause the processing system 200 to carry out the method according to any one of the embodiments described. For example, the computer program product is stored on the memory 206.

In an embodiment, there is provided a computer-readable storage medium comprising instructions which, when executed by the processing system 200, cause the processing system 200 to carry out the method according to any one of the embodiments described. For example, the computer-readable storage medium is implemented as the memory 206.

It will be understood that the disclosed methods are computer-implemented methods. As such, there is also proposed a concept of a computer program comprising code means for implementing any described method when said program is run on a processing system 200.

The skilled person would be readily capable of developing a processing system 200 for carrying out any herein described method. Thus, each step of each embodiment may represent a different action performed by a processing system 200, and may be performed by a respective module of the processing system 200.

The processing system 200 can be implemented in numerous ways, with software and/or hardware, to perform the various functions required. The processing system 200 may employ one or more microprocessors that may be programmed using software (e.g. microcode) to perform the required functions. The processing system 200 may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system 200 may be embodied as a digital and/or analog processing system.

The memory 206 may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within the processing system 200, such that the one or more programs stored thereon can be loaded into a processing system 200.

Functions implemented by the processing system 200 may be implemented by a single processor or by multiple separate processing units which may together be considered to constitute the processing system 200. Such processing units may in some cases be remote from each other and communicate with each other in a wired or wireless manner.

The computer-readable storage medium comprises, for example, an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to an advantage. Any reference signs in the claims should not be construed as limiting the scope.

## Claims

1. A method of acoustic monitoring of a subject (110), the method comprising;
receiving acoustical data obtained in the vicinity of the subject (110);
identifying (301) a first portion (321) of the acoustical data representing acoustic events;
identifying (301) a second portion (324) of the acoustical data representing no acoustic events;
providing samples (322) based on the first portion (321) of the acoustical data to a classifier (300);
creating classified samples (320) by classifying the samples (322) with the classifier (300),
wherein each classified sample (320) is classified by the classifier (300) as a first class (311) or a second class (312),
wherein the first class (311) represents a respiratory acoustic event related to a condition of the respiratory system of the subject (110),
wherein the second class (312) does not represent the respiratory acoustic event;
determining a property (325, 625) of at least one of
the second portion (324) of the acoustical data, and
the classified samples (320);
performing a comparison (326) of the property (325, 625) with a reference property (306, 606);
wherein the reference property (306, 606) is of at least one of acoustical training data used to train the classifier (300) during a training phase, and
at least one classified training sample created by the classifier (300) based on the training acoustical data during the training phase;
generating a similarity metric (307) based on the comparison (326), wherein the similarity metric (307) is representative of similarity between the property (325, 625) and the reference property (306, 606).

2. The method of claim 1, comprising:
creating with the classifier (300) the classified samples (320) with a probability of being the first class (311),
classifying with the classifier (300) a sample as the first class (311) if the probability is above a threshold,
classifying with the classifier (300) a sample as the second class (312) if the probability is below the threshold,
determining the property (625) based on the probability of at least one classified sample.

3. The method of claim 2, wherein the property (625) is based on a distribution of the probabilities of at least a subset of the classified samples.

4. The method of claim 3, wherein the reference property (606) is based on a distribution of probabilities of at least a subset of the classified training samples.

5. The method of any one of claims 2-4, comprising:
receiving multiple sets of acoustical data, each set of acoustical data representing a different time interval;
for each set of acoustical data:
creating classified samples (320) with the classifier (300), summing the probability of the classified samples, and counting a number of classified samples (320) in the first class (311);
determining a correlation (804) between the number of classified samples (320) in the first class (311) and the sum of the probability, based on the multiple sets of acoustical data;
determine as the property (625) a deviation between the sum of the probabilities and the correlation (804) for each set of acoustical data.

6. The method of any one of the preceding claims,
wherein the acoustical training data comprises a first portion and a second portion,
wherein the first portion of the acoustical training data is representative of acoustic events,
wherein the second portion of the acoustical training data is representative of no acoustic events,
wherein the reference property (306) is based on a characteristic of at least the second portion (324) of the acoustical training data.

7. The method of claim 6, wherein the characteristic comprises one of an average sound level, spectral tilt, a number of acoustic events, and a temporal distance between acoustic events.

8. The method of any one of the preceding claims, comprising, in case the similarity exceeds a similarity threshold, indicating with the similarity metric (307) a change in an environment of the subject (110) between when the acoustical data was obtained and when the acoustical training data was obtained.

9. The method of claim 8, comprising suggesting with the similarity metric (307) to retrain the classifier (300) for the change in the environment, in case the similarity exceeds the similarity threshold.

10. The method of any one of the preceding claims, comprising:
receiving (901) acoustical training data obtained in the vicinity of the subject (110);
identifying (902) a first portion of the acoustical training data representing acoustic events;
identifying (903) a second portion of the acoustical training data representing no acoustic events;
providing (904) training samples based on the first portion of the acoustical training data to the classifier (300);
creating (905) the classified training samples by classifying the training samples with the classifier (300),
wherein each classified training sample is classified by the classifier (300) as the first class (311) or the second class (312);
providing at least a subset of the classified training samples for annotation;
receiving the annotation;
training the classifier (300) based on the annotation.

11. The method of claim 10, comprising:
creating (1000) snippets from the first portion of the acoustical training data;
providing (1002) the snippets for annotation.

12. The method of any one of the preceding claims, wherein the condition of the respiratory system comprises coughing,
wherein the first class (311) represents a coughing event;
wherein the second class (312) does not represent a coughing event.

13. A computer program product, comprising instructions which, when executed by a processing system (200), cause the processing system (200) to carry out the method of any one of claims 1-12.

14. An acoustic monitoring system (100), comprising:
a processing system (200) configured to perform the method of any one of claims 1-12; and
an acoustic sensor (102) for generating the acoustical data representative of acoustic events related to the condition of the respiratory system of the subject (110),
wherein the processing system (200) is coupled to the acoustic sensor (102) to receive the acoustical data.

15. A computer-readable storage medium (206) comprising instructions which, when executed by a processing system (200), cause the processing system (200) to carry out the method of any one of claims 1-12.
